# EUROPEAN PATENT APPLICATION

(11) **EP 3 878 472 A1**
(43) Date of publication of application: **15.09.2021**
(21) Application number: 20162690.0
(22) Date of filing: 12.03.2020
(51) Int. Cl.: A61K 47/54, A61K 47/55, A61P 35/00

(54) **PROTEOLYSIS TARGETING CHIMERA (PROTAC) FOR DEGRADATION OF AURORA A-KINASE**

(71) Applicant: Julius-Maximilians-Universitaet Wuerzburg, 97070 Wuerzburg (DE)
(72) Inventor: WOLF, Elmar, 97084 Würzburg (DE); EILERS, Martin, 97074 Würzburg (DE)
(74) Representative: Dr. Gassner & Partner mbB

(57) **Abstract**

The invention concerns a Proteolysis Targeting Chimera (PROTAC) or a pharmaceutically acceptable salt thereof for degradation of Aurora A-kinase in cells of a mammal which PROTAC has the chemical structure AAB-L-E3B, wherein AAB is a binding unit for Aurora A-kinase, L is a linker and E3B is a binding unit for E3-ubiquitin ligase Cereblon, wherein E3B comprises the structure of thalidomide or of one of its analogs lenalidomide, pomalidomide, and apremilast, wherein L comprises or consists of an alkyl ether residue or a polyalkyl ether residue which polyalkyl ether residue has at least two ether groups or at least two ether groups in which one O-atom is replaced by an S-atom or a part of the O-atoms is replaced by S-atoms or wherein L comprises or consists of an alkyl thioether residue or a polyalkyl thioether residue which polyalkyl thioether residue has at least two thioether groups, wherein L connects AAB and E3B via a chain of atoms having five to seventeen subsequently arranged atoms, wherein atoms of functional groups connecting the linker with AAB and E3B are not considered as part of said chain.

## Description

The invention concerns a Proteolysis Targeting Chimera (PROTAC) for degradation of Aurora A-kinase or a pharmaceutically acceptable salt thereof. The PROTAC has the chemical structure AAB-L-E3B, wherein AAB is a binding unit for Aurora A-kinase, L is a connecting unit and E3B is a binding unit for E3-ubiquitin ligase Cereblon.

US 2019/0210996 A1 discloses a compound of formula "target protein binder-linker-Cereblon binder" or a pharmaceutically acceptable salt thereof, wherein the target protein is one of several kinases, i. a., Aurora A-kinase. The linker is a chemical linker group which is four to twenty atoms in shortest length. It may be a straight chain alkylene group of four to twenty carbon atoms in which one or more carbon atoms is replaced by a group independently selected from -O-, -NH-, -N(CH₃)-, -CO-, piperidine, piperazine, pyrimidine and pyridine. The Cereblon binding moiety may be thalidomide, pomalidomide or lenalidomide. US 2019/0210996 A1 further discloses a method of degrading the target protein comprising administering to a human in need thereof a therapeutically effective amount of the compound or a pharmaceutically acceptable salt thereof.

From WO 2017/201449 A1 antibody-PROTAC conjugates are known. The PROTAC comprises an E3 ligase binding group covalently bound to a linker which is covalently bound to a protein binding group. The E3 ligase binding group may be a group that binds an E3 ligase selected from a group comprising Cereblon. The E3 ligase binding group may be selected from a group comprising, i. a., thalidomide, lenalidomide and pomalidomide. The protein binding group may be a group that binds among a lot of other proteins Aurora A.

Wang, Y. et al., Acta Pharm Sin B, 2020, 10(2), pages 207 to 238 concerns degradation of proteins by PROTACs and other strategies. It discloses Cereblon-based PROTACs comprising a linker and a binding unit for E3-ubiquitin ligase Cereblon which binding unit may be thalidomide, lenalidomide or pomalidomide.

The problem to be solved by the present invention is to provide an alternative PROTAC, a method for synthesizing that PROTAC and said PROTAC for use in a medical treatment.

The problem is solved by the features of claims 1, 11 and 14. Embodiments of the invention are subject-matter of claims 2 to 10, 12, 13 and 15.

According to the invention a Proteolysis Targeting Chimera (PROTAC) for degradation of Aurora A-kinase in cells of a mammal or a pharmaceutically acceptable salt of said PROTAC is provided. The PROTAC has the chemical structure AAB-L-E3B, wherein AAB is a binding unit for Aurora A-kinase, L is a linker and E3B is a binding unit for E3-ubiquitin ligase Cereblon, wherein E3B comprises the structure of thalidomide or one of its analogs lenalidomide, pomalidomide, and apremilast. For comprising the structure of thalidomide or one of said analogs, thalidomide or the analog may be coupled to a coupling moiety facilitating coupling to L such as in 2-((2-(2,6-dioxopiperidin-3-yl)-1,3-dioxoisoindolin-4-yl)oxy)acetic acid (compound a):

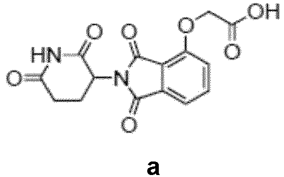

L comprises or consists of an alkyl ether residue or a polyalkyl ether residue which polyalkyl ether residue has at least two ether groups in which one O-atom is replaced by an S-atom or a part of the O-atoms is replaced by S-atoms. Alternatively, it is possible that L comprises or consists of an alkyl thioether residue or a polyalkyl thioether residue which polyalkyl thioether residue has at least two thioether groups. L connects AAB and E3B via a chain of atoms having five to seventeen subsequently arranged atoms, wherein atoms of functional groups connecting the linker with AAB and E3B are not considered as part of said chain. This means that no linear sequence of atoms of the alkyl ether residue, the polyalkyl ether residue, alkyl thioether residue, or the polyalkyl thioether residue exceeds the number of 17 atoms. Functional groups connecting the linker with AAB and E3B may be -NH-, -COO-, -CONH-, -CSO- or -COS-.

The inventors of the present invention have recognized that the length of the linker is essential for the function of the PROTAC. Further they have recognized that hydrophilicity provided by the ether group(s) or thioether group(s) is important for the function as a PROTAC. Furthermore, they have recognized that a binding unit for another E3-ubiquitin ligase than Cereblon, e. g. von Hippel-Lindau tumor suppressor (VHL), does not result in a functional PROTAC. The inventors assume that the specific spatial relation between Cereblon and Aurora A that is achieved with the specific linker in the PROTAC according to the invention is important for causing the depletion of Aurora A by the PROTAC.

L may be any of the following moieties:
1. -CH₂-CH₂-O-CH₂-CH₂-
2. -CH₂-CH₂-(CH₂-O-CH₂)₃-CH₂-CH₂-
3. -CH₂-(CH₂-O-CH₂)₂-CH₂-
4. -(CH₂)₃-
5. -(CH₂)₅-

In binding studies, the inventors found that the PROTACs according to the invention can bind to Aurora A but binding was reduced when the linker was short and connected AAB and E3B via a chain of only five subsequently arranged atoms. Further, the inventors found that the PROTAC according to the invention can form Aurora A-E3-ubiquitin ligase complexes in cells treated with the PROTAC according to the invention and that this PROTAC can achieve almost complete degradation of Aurora A in cells treated with said PROTAC. Degradation was observed in lymphoblasts of a human leukemia cell line and cells of human neuroblastoma, hepatocellular carcinoma and osteosarcoma cell lines. Furthermore, it was observed that depletion of Aurora A by the PROTAC induced apoptosis in all tested cells of cancer cell lines.

L may be bound to AAB via an amide bond A, in particular a peptide bond A. Alternatively or in addition L may be bound to E3B via an amide bond B, in particular a peptide bond B. Denomination of the amide bonds and the peptide bonds with "A" and "B" has only been chosen for differentiation between the binding of L to AAB and the binding of L to E3B. However, amide bond A as such may be identical with amide bond B as such and peptide bond A as such may be identical with peptide bond B as such. If E3B comprises the structure of lenalidomide or pomalidomide, L may be bound to this structure by use of lenalidomide's or pomalidomide's amino group.

An H-atom of the amide bond A may be substituted by an alkyl residue A, in particular a methyl residue A or an ethyl residue A. Alternatively or in addition an H-atom of the amide bond B may be substituted by an alkyl residue B, in particular a methyl residue B or an ethyl residue B. This stabilizes the amide bond(s) of the PROTAC against degradation, in particular by hydrolyzes. As above, denomination of the methyl residues and the ethyl residues with "A" and "B" has only been chosen for differentiation of residues at amide bond A and at amide bond B though the structures of methyl residue A and methyl residue B as well as the structures of ethyl residue A and ethyl residue B are identical.

In an embodiment of the invention L comprises at least two O-atoms, at least two S-atoms or at least one O-atom and at least one S-atom. This results in a higher hydrophilicity than that of a PROTAC in which L comprises only one O-atom or only one S-atom. A higher hydrophilicity seems to improve efficiency of the PROTAC with respect to the degradation of Aurora A-kinase. Furthermore, efficiency seems to be improved when L is a linear molecule residue and/or said chain of atoms has six to thirteen subsequently arranged atoms, in particular six to ten subsequently arranged atoms.

One or more C - C bonds of L may be replaced by a C = C double bond for increasing rigidity of the linker.

In an embodiment the alkyl ether residue, polyalkyl ether residue, alkyl thioether residue or polyalkyl thioether residue of the linker L is substituted at least at one position by an amino group, a hydroxyl group or a carbonyl group. It seems to be that these groups stabilize the complex of Aurora A and Cereblon with the PROTAC.

AAB may comprise the structure of alisertib, (3-chloro-2-fluorophenyl)[4-[[6-(2-thiazolylamino)-2-pyridinyl]methyl]-1-piperazinyl]-methanone (MK-8745) or 1-[4-({4-[(5-cyclopentyl-1H-pyrazol-3-yl)amino]pyrimidin-2-yl}amino)phenyl]-3-[3-(trifluoromethyl)phenyl]urea (CD532).

If AAB comprises the structure of alisertib this structure may be bound to L via a peptide bond formed from alisertib's carboxy group and an amino group of L.

In a specific embodiment the PROTAC is any of the following compounds:

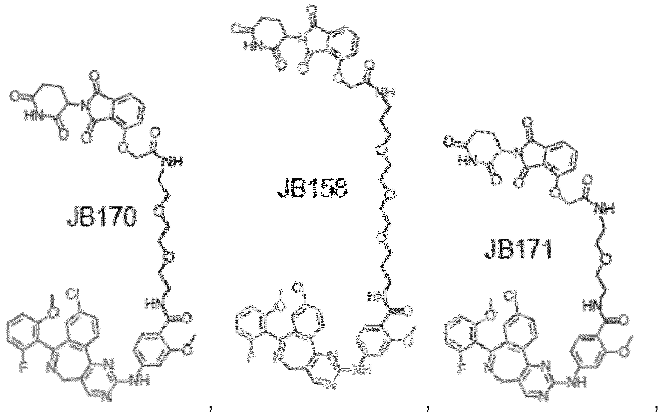

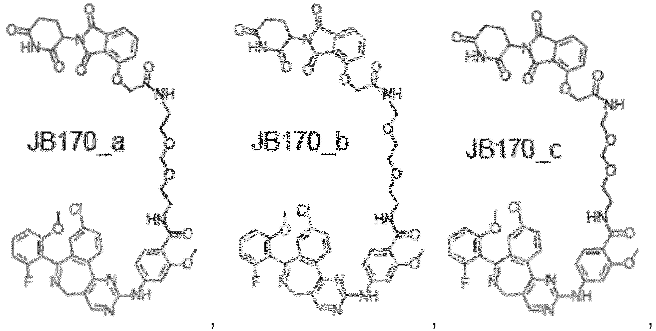

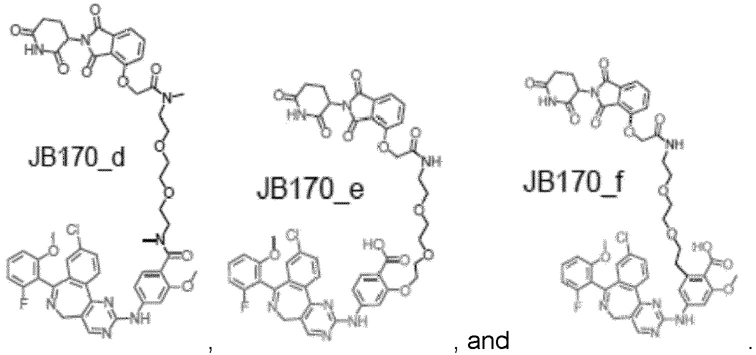

The invention further concerns a method for synthesizing the PROTAC, wherein 2-((2-(2,6-dioxopiperidin-3-yl)-1,3-dioxoisoindolin-4-yl)oxy)acetic acid (compound **a**) 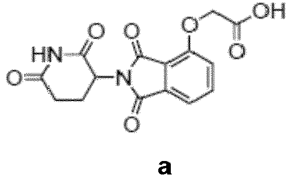 is dissolved in an aprotic, in particular organic, solvent, in particular under protective gas atmosphere, followed by addition of (1-[Bis(dimethylamino)methylene]-1H-1,2,3-triazolo[4,5-b]pyridinium 3-oxide hexafluorophosphate (HATU) and N,N-diisopropylethylamine (DIPEA) or trimethylamine as well as a linker precursor having the structure "NH2-alkylether residue-NH-amine protecting group", "NH2-polyalkylether residue-NH-amine protecting group", "NH2-alkyl thioether residue-NH-amine protecting group" or "NH2-polyalkyl thioether residue-NH-amine protecting group" and further followed by incubation resulting in an intermediate product which is then deprotected to result in compound **b** 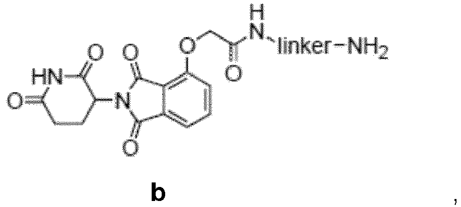 wherein the polyalkyl ether residue has at least two ether groups or at least two ether groups in which at least one O-atom is replaced by an S-atom and the polyalkyl thioether residue has at least two S-atoms, wherein the alkyl ether residue, the polyalkyl ether residue, alkyl thioether residue, or the polyalkyl thioether residue has a chain of atoms having five to seventeen, in particular six to thirteen, in particular six to ten, subsequently arranged atoms, wherein no linear sequence of atoms of the alkyl ether residue, the polyalkyl ether residue, alkyl thioether residue, or the polyalkyl thioether residue exceeds the number of seventeen atoms, wherein compound **b** is dissolved in an aprotic, in particular organic, solvent, in particular under protective gas atmosphere, followed by addition of (1-[Bis(dimethylamino)methylene]-1H-1,2,3-triazolo[4,5-b]pyridinium 3-oxide hexafluorophosphate (HATU), alisertib and N,N-diisopropylethylamine (DIPEA) or trimethylamine and further followed by incubation to result in compound **c** 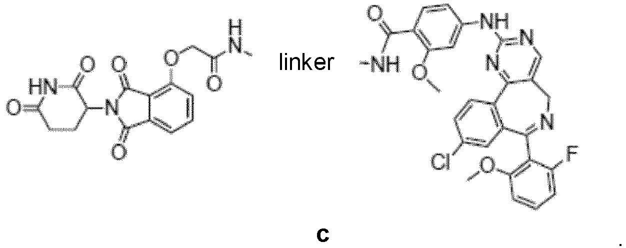 2-((2-(2,6-dioxopiperidin-3-yl)-1,3-dioxoisoindolin-4-yl)oxy)acetic acid may be synthesized as described in the "Supporting Information" concerning Remillard, D. et al., Angew. Chem. Int. Ed. 2017, 56, pages 5738 to 5743. The protective gas may be argon or nitrogen. The pH value during the first and the second incubation may be checked to be higher than 12 and if it is not higher than 12 it may be adjusted to a value higher than 12. The solvent may be a polar solvent, in particular Dimethylformamide (DMF). A solvent is considered as polar if its dielectric constant is higher than 15. The amine protecting group may be ter-butyloxycarbonyl (Boc).

The linker precursor may be any of the following compounds **1** to **5**:
1. NH₂-CH₂-CH₂-O-CH₂-CH₂-NH-Boc
2. NH₂-CH₂-CH₂-(CH₂-O-CH₂)₃-CH₂-CH₂-NH-Boc
3. NH₂-CH₂-(CH₂-O-CH₂)₂-CH₂-NH-Boc
4. NH₂-(CH₂)₃-NH-Boc
5. NH₂-(CH₂)₅-NH-Boc.

Deprotection may be achieved as known in the art, e. g., by purifying the intermediate product and transferring the intermediate product to acidic conditions, in particular by dissolving it in a mixture of dichloromethane and trifluoroacetic acid.

The invention further concerns the PROTAC according to the invention for use in the treatment of cancer of a human being or another mammal. The cancer may be leukemia, neuroblastoma, hepatocellular carcinoma or osteosarcoma.

Embodiments of the invention:
- Fig. 1: shows schematically the general synthesis of compounds JB158, JB159, JB169, JB170, JB171 having a thalidomide moiety and of compounds JB160, JB161 having a VHL-ligand which compounds are linked to alisertib by various (poly)ethylene glycol (PEG) and aliphatic linkers.
- Fig. 2: shows an immunoblot and quantification of endogenous Aurora-A from cells of human leukemia cell line MV4-11 which cells were treated in each case with a single dose of compounds JB158, JB160, JB161, JB169, JB170, JB159 and JB171 in a concentration of 1 µM. Vinculin served as a loading control in the immunoblot.
- Fig. 3: shows immunoblots of endogenous Aurora-A from MV4-11 untreated cells and cells treated with various concentrations of JB170 (upper panel) and JB158 (lower panel) as well as with unconjugated alisertib (uA) for 6 hours with Vinculin as a loading control.
- Fig. 4: shows immunoblots of endogenous Aurora-A from cells of human osteosarcoma cell line U2OS treated with various concentrations of JB170 (upper panel) and JB158 (lower panel) as well as with unconjugated alisertib (uA) for 6 hours with Vinculin as a loading control.
- Fig. 5: shows immunoblots of endogenous Aurora-A from cells of human hepatocellular carcinoma cell line HLE treated with various concentrations of JB170 (upper panel) and JB158 (lower panel) as well as with unconjugated alisertib (uA) for 6 hours with Vinculin as a loading control.
- Fig. 6: shows an immunoblot of endogenous Aurora-A from cells of human neuroblastoma cell line IMR5 treated with JB170 in a concentration of 0.1 µM for the indicated time periods with Vinculin as a loading control.
- Fig. 7: shows an immunoblot of endogenous Aurora-A from cells of HLE cells treated with JB158 in a concentration of 0.1 µM for the indicated time periods with Vinculin as a loading control.
- Fig. 8: is a bar diagram showing cellular viability of MV4-11 cells treated with 1 µM JB170.
- Fig. 9: is a diagram showing apoptosis of MV4-11 cells treated with 0.5 µM JB170 and stained for Annexin and with Propidium Iodide (PI), wherein the amounts of early (Annexin+, PI-) and late (Annexin+, PI+) apoptotic cells were analyzed by FACS.
- Fig. 10: Diagram showing apoptosis of IMR5 cells (EtOH) and IMR5 cells expressing Aurora-A^{T217D} upon incubation with doxycycline (Dox (Aurora-A^{T217D})) which cells were treated with 0.5 µM JB170 for 72 hours, stained for Annexin and with Propidium Iodide (PI) and analyzed by FACS.

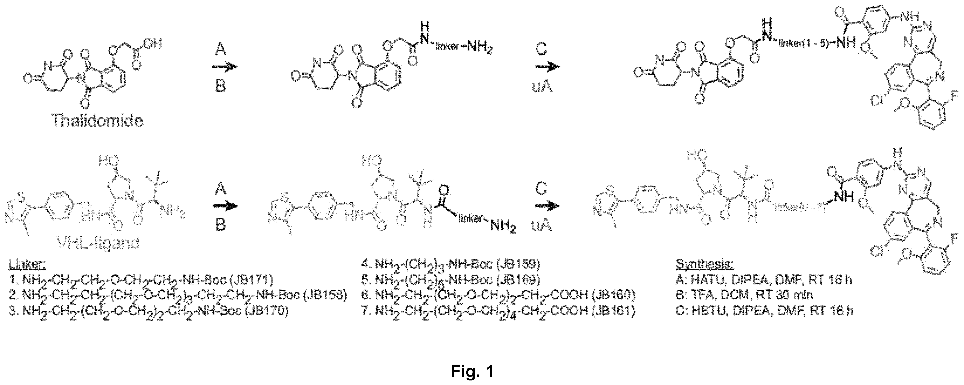

The inventors synthesized Aurora-A targeting chimeric degraders by linking alisertib to thalidomide as a Cereblon-binding moiety or to a HIF1-derived peptidomimetic as a VHL-binding moiety via a variety of (poly)ethylene glycol (PEG) or aliphatic linkers as schematically shown in Fig. 1.

In detail, syntheses were performed as follows:
Unless otherwise stated, all reactions were performed at room temperature (RT). The Structure and purity of the compounds was confirmed by HPLC, mass spectrometry and NMR.

For thalidomide derivative-linker coupling the thalidomide-derivative 2-((2-(2,6-dioxopiperidin-3-yl)-1,3-dioxoisoindolin-4-yl)oxy)acetic acid (Compound **a**) (310 mg, 0.93 mmol, 1.0 eq) was dissolved in 5 ml DMF under argon and HATU (418 mg, 1.02 mmol, 1.1 eq) and DIPEA (324 µl, 1.86 mmol, 2.0 eq) were added. After 15 minutes stirring at RT, a solution of the respective linker **1-5** (1.12 mmol, 1.2 eq) in 2 ml DMF was added and the reaction mixture was stirred overnight. The mixture was transferred into a separation funnel and extracted with water (20 ml) and ethyl acetate (30 ml). The layers were separated and the aqueous layer was extracted 3 times with ethyl acetate. The organic layers were combined, dried over MgSO₄ and concentrated. The residue was purified with Flash Chromatography (FC) using DCM/MeOH, 95:5. The resulting intermediate product was obtained as colorless oil. The intermediate product was dissolved in a mixture of DCM and trifluoroacetic acid (6 ml, 40 vol.%). After 30 min the solvent was removed under reduced pressure, to obtain the deprotected thalidomide-linker intermediate as TFA-salt (**c-g**). The reaction is illustrated in the following reaction

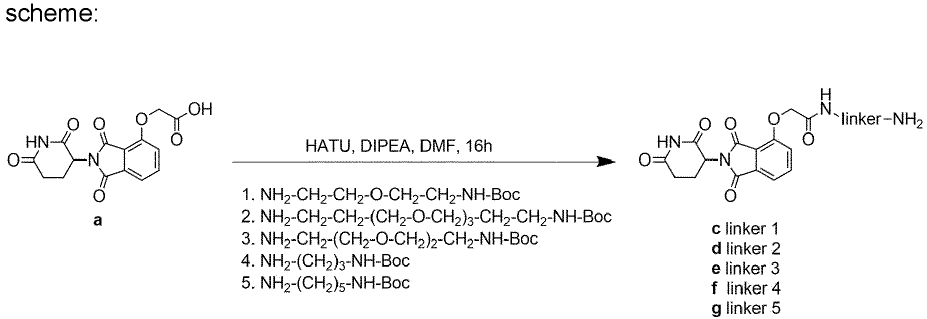

Intermediate **c** *N*-(2-(2-aminoethoxy)ethyl)-2-((2-(2,6-dioxopiperidin-3-yl)-1,3-dioxoisoindolin-4-yl)oxy)acetamide (Th-PEG1-NH2) was obtained with overall yield of 69% (339 mg, 0.93 mmol) over two steps (1^{st} 71%, 2^{nd} 97%).

Intermediate **d** *N*-(3-(2-(2-(3-Aminopropoxy)ethoxy)ethoxy)propyl)-2-((2-(2,6-dioxopiperidin-3-yl)-1,3-dioxoisoindolin-4-yl)oxy)acetamide (Th-PEG3-NH2) was obtained with overall yield of 57% (285 mg, 0.53 mmol) over two steps (1^{st} 60%, 2^{nd} 95%).

Intermediate **e** *N*-(2-(2-(2-aminoethoxy)ethoxy)ethyl)-2-((2-(2,6-dioxopiperidin-3-yl) -1,3-dioxoisoindolin-4-yl)oxy)acetamide (Th-PEG2-NH₂) was obtained with overall yield of 73% (316 mg, 0.68 mmol) over two steps (1^{st} 75%, 2^{nd} 97%).

Intermediate **f** *N*-(3-aminopropyl)-2-[[2-(2,6-dioxo-3-piperidinyl)-2,3-dihydro-1,3-dioxo-1H-isoindol-4-yl]oxy] acetamide was obtained with overall yield of 60% (233 mg, 0.60 mmol) over two steps (1^{st} 63%, 2^{nd} 95%).

Intermediate **g** *N*-(5-aminopentyl)-2-[[2-(2,6-dioxo-3-piperidinyl)-2,3-dihydro-1,3-dioxo-1H-isoindol-4-yl]oxy] acetamide was obtained with overall yield of 53% (206 mg, 0.50 mmol) over two steps (1^{st} 60%, 2^{nd} 88%).

For VHL-ligand-linker coupling VHL ligand Compound **b** (92 mg, 0.214 mmol, 1.0 eq) was dissolved in 6 ml DMF in a flask (A) (reaction mixture (A)) under argon. The respective linker **6-7** (0.236 mmol, 1.1 eq) and HATU (92 mg, 0.241 mmol, 1.1 eq) were dissolved in 6 ml DMF in a separate flask (B) (reaction mixture (B)) under argon atmosphere. Both flasks were cooled to 0 °C. DIPEA (117 µl, 0.65 mmol, 4.0 eq) was added to flask (A), 0.12 ml (117 µl, 0.65 mmol, 4 eq) to flask (B). The ice bath was removed and both solutions were stirred for 20 min, then solution from flask (B) was transferred into flask (A) and reaction mixtures was stirred overnight. The orange mixture was transferred into a separation funnel and brine was added. The layers were separated and the aqueous layer was extracted 5 times with DCM. The organic layers were combined, dried over MgSO₄, filtered and concentrated under reduced pressure. The remaining residue was purified with FC (DCM/MeOH, 100:0-95:5). The resulting intermediate product was obtained as colorless oil. The intermediate product was dissolved in DCM and trifluoroacetic acid (10 vol.%) was added. After 30 min the solvent was removed under reduced pressure, to obtain the deprotected VHL-ligand-linker intermediate as free TFA salt (**h-i**). The reaction is illustrated in the following reaction scheme:

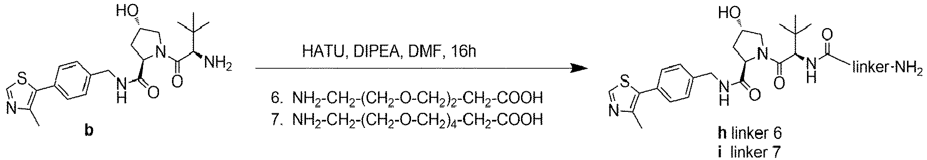

Intermediate **h** L-Prolinamide, *N*-[3-[2-(2-aminoethoxy)ethoxy]-1-oxopropyl]-3-methyl-*L*-valyl-4-hydroxy-*N*-[[4-(4-methyl-5-thiazolyl)phenyl]methyl]-(4*R*) (VHL-PEG2-NH₂)) was obtained with overall yield of 59% (75 mg, 0.13 mmol) over two steps (1^{st} 61%, 2^{nd} 97%).

Intermediate **i** *N*-[3-[2,2[2-aminoethoxy-(2-aminoethoxy)]ethoxy]-1-oxopropyl]-3-methyl-*L*-valyl-4-hydroxy-*N*-[[4-(4-methyl-5-thiazolyl)phenyl]methyl]-(4R) (VHL-PEG4-NH₂) was obtained with overall yield of 52% (75 mg, 0.11 mmol) over two steps (1^{st} 51%, 2^{nd} 98%).

For coupling intermediate compounds **c-g** (thalidomide derivatives) and **h-i** (VHL-ligands), 0.02 mmol (1.0 eq) were dissolved in 1.5 ml DMF. HBTU (7.6 mg, 0.02 mmol, 1.0 eq) was added. After dissolving, alisertib (10 mg, 0.02 mmol, 1.0 eq) was added, followed by DIPEA (14 µl, 0.08 mmol, 4.0 eq). pH was adjusted to 8-10 by adding more equivalents DIPEA. Reaction mixtures were stirred overnight. The solvent was removed using a SpeedVac concentrator. The residues were purified and characterized via preparative and analytical HPLC. All reactions were done with 10 mg alisertib except JB170. This compound was synthesized first with 5 mg and then with 50 mg alisertib. The reaction is illustrated in the following reaction scheme: 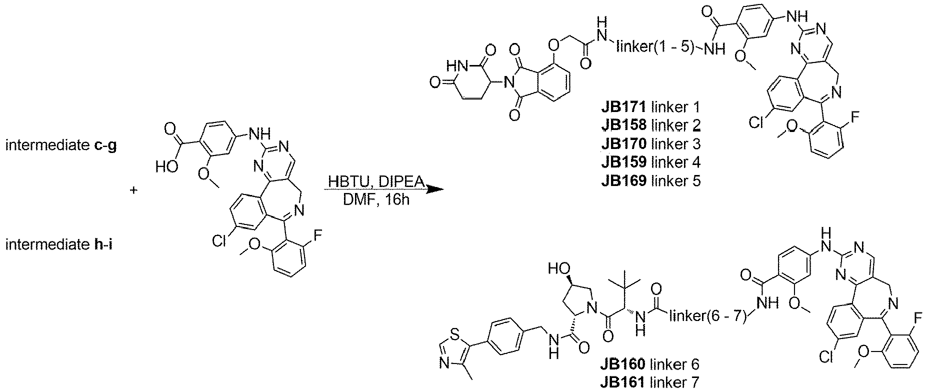 JB158 thalidomide-PEG3-alisertib was obtained as white solid with a yield of 90% (17 mg, 0.018 mmol).

JB159 thalidomide-(CH₂)₃-alisertib was obtained as white solid with a yield of 70% (12 mg, 0.013 mmol).

JB160 VHL-PEG2-alisertib was obtained as white solid with a yield of 43% (9 mg, 0.008 mmol).

JB161 VHL-PEG4-alisertib was obtained as white solid with a yield of 62% (14 mg, 0.012 mmol).

JB169 thalidomide-(CH₂)₅-alisertib was obtained as white solid with a yield of 61% (11 mg, 0.012 mmol).

JB170 thalidomide-PEG2-alisertib was obtained as white solid with a yield of 48% (9 mg, 0.009 mmol).

JB171 thalidomide-PEG1-alisertib was obtained as white solid with a yield of 75% (13 mg 0,014 mmol).

### Formation of productive Aurora-A-E3 ligase complexes

For testing which of the synthesized molecules mediate the formation of Aurora-A-E3 ligase complexes resulting in degradation of endogenous Aurora-A, cells of the leukemia cell line MV4-11 were treated with 0.1 µM thalidomide-based PROTACs JB158, JB160, JB161, JB169 or JB170 or 1 µM VHL-ligand-based PROTACs JB159 or JB171 at a single dose of the respective compound. Then, the cells were lysed in RIPA lysis buffer (50 mM HEPES pH 7.9, 140 mM NaCl, 1 mM EDTA, 1% Triton X-100, 0.1% SDS, 0.1% sodium deoxycholate) containing protease and phosphatase inhibitors (Sigma) and incubated for 20 minutes at 4 °C in head-over-tail. Lysates were cleared by centrifugation. Protein quantification was done using BCA assay and equal amounts of proteins were separated by BisTris-PAGE and transferred to PVDF membranes (Millipore). The membranes were blocked with 5% (w/v) nonfat dry milk dissolved in TBS-T (20 mM Tris/HCI, pH 7.5, 150 mM NaCl, and 0.1% (v/v) Tween 20) at RT for 1 hour and then incubated with the primary antibody overnight at 4 °C. Visualization was done with HRP-labeled secondary antibodies and detected using Chemiluminescent HRP substrate (Millipore) in LAS3000 or LAS4000 Mini (Fuji). The signal was quantified using ImageJ (version 1.52q) or Image Studio Lite (LI-COR Biosciences, Version 5.2.5).

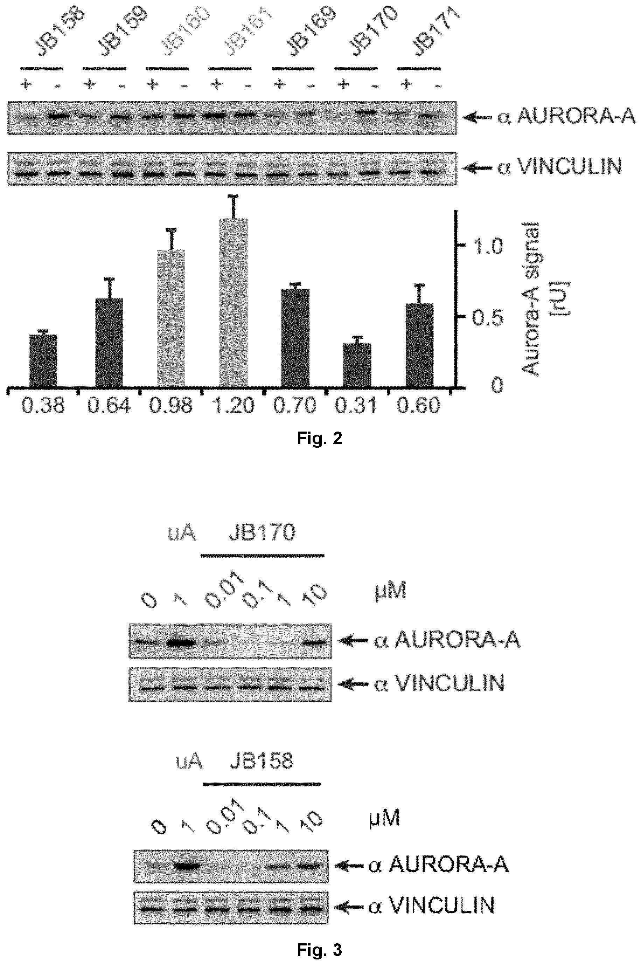

The upper part of Fig. 2 shows immunoblots of Aurora-A. Vinculin was used as a loading control. The bar diagram at the lower part of Fig. 2 shows cellular Aurora-A levels of the cells upon degrader treatment compared to the Aurora A level of control cells. Error bars represent standard deviations of four biological replicate experiments.

While neither of the VHL-ligand-based degraders JB160 and JB161 reduced Aurora-A steady-state levels, thalidomide-based degrader JB158 resulted in a reduction of Aurora-A protein level by 62% and thalidomide-based degrader JB170 in a reduction by 69%. In these two degraders resulting in the strongest Aurora-A degradation the linkage between the structure of thalidomide and that of alisertib is provided by a linker having two (JB170) or three (JB158) ethylene glycol moieties. Both compounds led to a rapid decrease in Aurora-A levels that reached its maximum after about three hours of treatment with JB158 or JB170.

### Degradation of endogenous Aurora-A

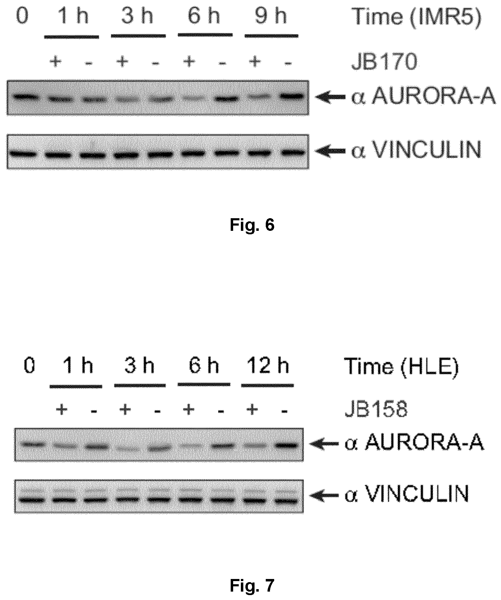

Cells of cell lines MV4-11, U2OS and HLE were treated with indicated concentration of compounds JB170 or JB158 as well as with unconjugated alisertib (uA) for 6 hours. In further experiments cells of cell lines IMR5 and HLE were treated with 0.1 µM of compound JB170 for the indicated time periods. Afterwards, cells were lysed in RIPA lysis buffer (50 mM HEPES pH 7.9, 140 mM NaCl, 1 mM EDTA, 1% Triton X-100, 0.1% SDS, 0.1% sodium deoxycholate) containing protease and phosphatase inhibitors (Sigma) and incubated for 20 minutes at 4 °C in head-over-tail. Further processing of resulting cell lysates and immunoblotting was performed as described above. Results for cells of cell lines MV4-11, U2OS and HLE treated with JB170 (upper panel) and JB158 (lower panel) are shown in Figs. 3 to 5. Results for cells treated with 0.1 µM of JB170 for the indicated time periods are shown for cells of cell lines IMR5 in Fig. 6 and for cells of cell lines HLE in Fig. 7.

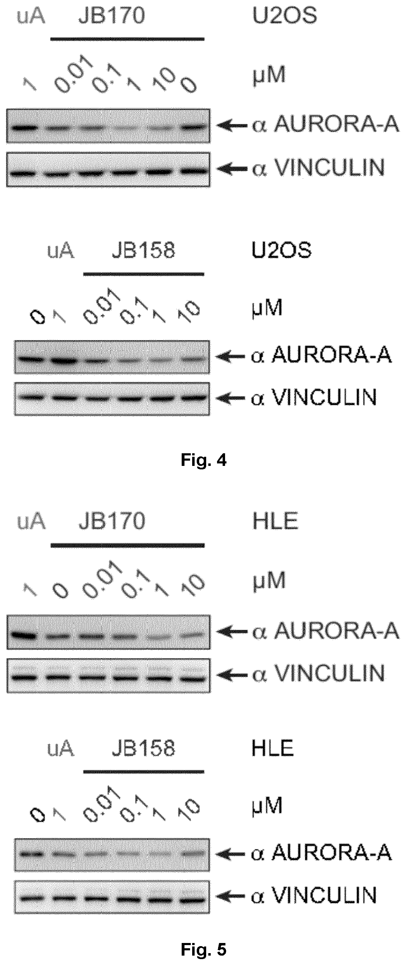

According to Fig. 3, upper panel Aurora-A levels already significantly decreased in MV4-11 cells at concentrations of 10 nM JB170 and almost complete degradation was observed at 100 nM and 1 µM. In agreement with other degraders/PROTACs, a reversal of the Aurora-A depleting activity was observed at high concentrations of JB170. This phenomenon is called "Hook effect" and is expected for degrader molecules, which depend on ternary complex formation. 1 µM of non-conjugated alisertib did not decrease Aurora-A levels, but induced a significant increase, as frequently observed for type-1 kinase inhibitors that stabilize the active state of their targets. Similar results shown in Fig. 3, lower panel were obtained with JB158 and with U2OS cells (Fig. 4, upper panel with JB170 and lower panel with JB158) and HLE cells (Fig. 5, upper panel with JB170 and lower panel with JB158). The degree of depletion and the concentration, at which the Hook effect became visible varied significantly between cell lines, potentially due to different cellular concentrations of Cereblon and Aurora-A.

Figs. 6 and 7 show that maximal degradation of endogenous Aurora-A occurred in IMR5 cells and HLE cells already after 3 to 6 hours.

### Cell death caused by a PROTAC according to the invention

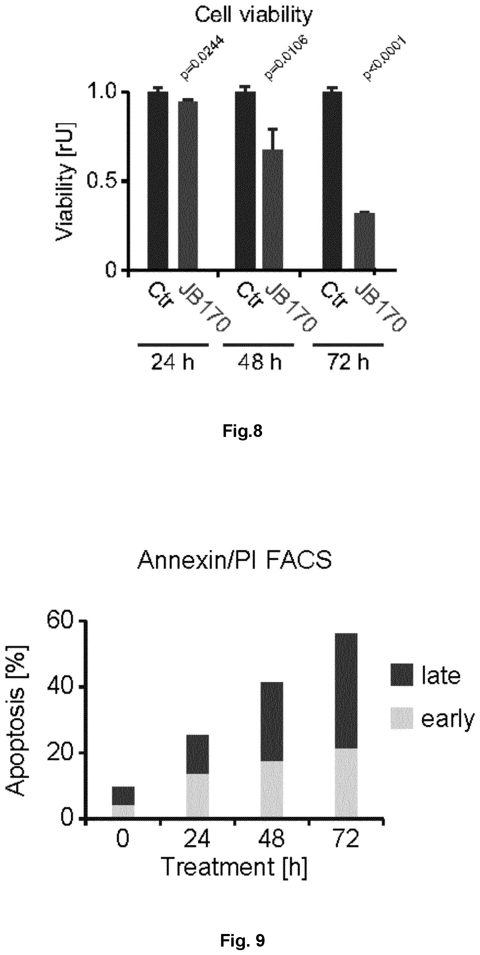

To determine the effect of JB170-mediated depletion of Aurora-A on cancer cell survival, MV4-11 cells were treated with JB170 and the intracellular reduction of resazurin to resorufin was measured to assess cell viability via an alamarBlue assay. For alamarBlue assay, 6000 MV4-11 cells per well were seeded into 96 well plate and treated with 1 µM JB170 for various time points (refreshed every 24 h). The alamarBlue assay (Thermo Fisher Scientific) was performed according to manufacturer's instruction using HS Cell Viability Reagent. The fluorescence was measured in Tecan Infinite-200 using a fluorescence excitation wavelength of 550 and an emission of 600 nm. After 72 hours, viable cells were reduced to 32% by JB170 compared to control cells (Fig. 8).

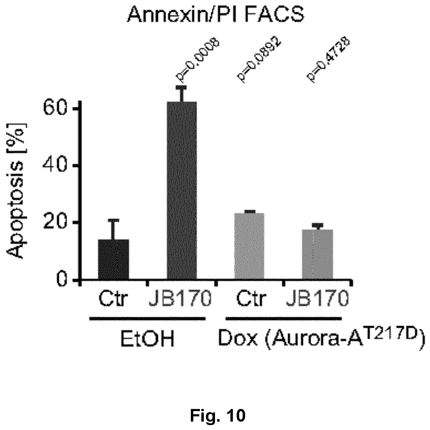

MV4-11 cells and IMR5 cells and IMR5 cells expressing Aurora-A^{T217D} upon incubation with doxycycline were treated with 0.5 µM JB170. For Annexin-PI FACS, the medium in which the cells were cultured was combined with the cells. The cells were washed once with ice-cold PBS, resuspended in 100 µl Annexin V Binding buffer (10 mM HEPES pH 7.4, 140 mM NaCl, 2.5 mM CaCl₂) with 2 µl of Annexin V/Pacific Blue dye and incubated for 15 minutes in the dark at room temperature. 400 µl Annexin V Binding buffer with propidium iodide (18.5 µM) was added and the samples were stored cold and in the dark until analysis. FACS experiments were performed on a BD FACSCanto II flow cytometer and analysis was done using BD FACSDIVA Software and FlowJo (version 8.8.6). Results are shown in Figs. 9 and 10.

Fig. 9 shows that JB170 increased the fraction of apoptotic cells in the culture over time, culminating in 56% of Annexin-positive MV4-11 cells after 72 hours.

Aurora-A^{T217D} is a functional mutant of Aurora-A. IMR5 cells express Aurora-A^{T217D} upon incubation with doxycycline (Dox) but not upon incubation with ethanol (control - EtOH). Affinity of Aurora-A^{T217D} to alisertib analogue MLN8054 is reduced vis-à-vis affinity of Aurora-A to that analogue by about a factor of 8.

Fig. 10 shows that expression of Aurora-A^{T217D} completely reverted the induction of apoptosis induced by JB170 in IMR5 cells indicating that JB170-induced apoptosis is exclusively caused by depletion of Aurora-A.

Experiments show that exposure of cancer cells to a PROTAC according to the invention resulted in strong induction of apoptosis and cytotoxicity in these cells.

## Claims

1. Proteolysis Targeting Chimera (PROTAC) or a pharmaceutically acceptable salt thereof for degradation of Aurora A-kinase in cells of a mammal which PROTAC has a chemical structure AAB-L-E3B, wherein AAB is a binding unit for Aurora A-kinase, L is a linker and E3B is a binding unit for E3-ubiquitin ligase Cereblon, wherein E3B comprises a structure of thalidomide or of one of its analogs lenalidomide, pomalidomide, and apremilast, wherein L comprises or consists of an alkyl ether residue or a polyalkyl ether residue which polyalkyl ether residue has at least two ether groups or at least two ether groups in which one O-atom is replaced by an S-atom or a part of the O-atoms is replaced by S-atoms or wherein L comprises or consists of an alkyl thioether residue or a polyalkyl thioether residue which polyalkyl thioether residue has at least two thioether groups, wherein L connects AAB and E3B via a chain of atoms having five to seventeen subsequently arranged atoms, wherein atoms of functional groups connecting the linker with AAB and E3B are not considered as part of said chain.

2. PROTAC according to claim 1, wherein L is bound to AAB via an amide bond A, in particular a peptide bond A, and/or wherein L is bound to E3B via an amide bond B, in particular a peptide bond B.

3. PROTAC according to claim 2, wherein an H-atom of the amide bond A is substituted by an alkyl residue A, in particular a methyl residue A or an ethyl residue A, and/or wherein an H-atom of the amide bond B is substituted by an alkyl residue B, in particular a methyl residue B or an ethyl residue B.

4. PROTAC according to any of the preceding claims, wherein L comprises at least two O-atoms, at least two S-atoms or at least one O-atom and at least one S-atom.

5. PROTAC according to any of the preceding claims, wherein L is a linear molecule residue and/or said chain of atoms has 6 to 13 subsequently arranged atoms, in particular 6 to 10 subsequently arranged atoms.

6. PROTAC according to any of the preceding claims, wherein one or more C - C bonds of L are replaced by a C = C double bond and/or wherein the alkyl ether residue, polyalkyl ether residue, alkyl thioether residue or polyalkyl thioether residue is substituted at least at one position by an amino group, a hydroxyl group or a carbonyl group.

7. PROTAC according to any of the preceding claims, wherein L is any of the following moieties:
-CH₂-CH₂-O-CH₂-CH₂-,
-CH₂-CH₂-(CH₂-O-CH₂)₃-CH₂-CH₂-,
-CH₂-(CH₂-O-CH₂)₂-CH₂-,
-(CH₂)₃-
and
-(CH₂)₅-.

8. PROTAC according to any of the preceding claims, wherein AAB comprises a structure of alisertib, (3-chloro-2-fluorophenyl)[4-[[6-(2-thiazolylamino)-2-pyridinyl]methyl]-1-piperazinyl]-methanone (MK-8745) or 1-[4-({4-[(5-cyclopentyl-1H-pyrazol-3-yl)amino]pyrimidin-2-yl}amino)phenyl]-3-[3-(trifluoromethyl)phenyl]urea (CD532).

9. PROTAC according to claim 8, wherein AAB comprises the structure of alisertib which is bound to L via a peptide bond formed from alisertib's carboxy group and an amino group of L.

10. PROTAC according to any of the preceding claims, wherein the PROTAC is any of the following compounds:

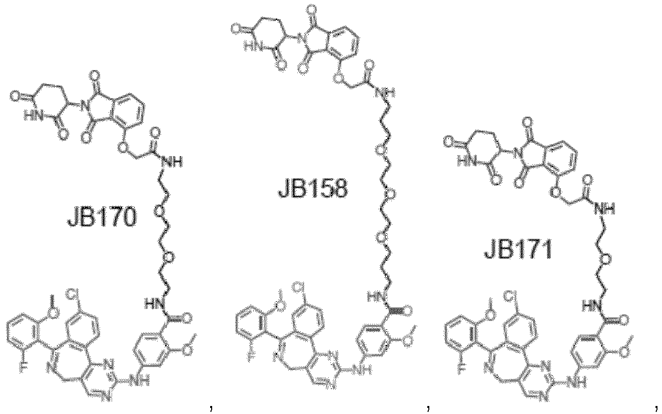

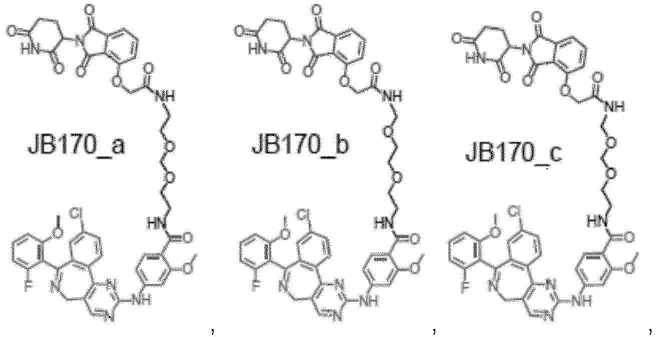

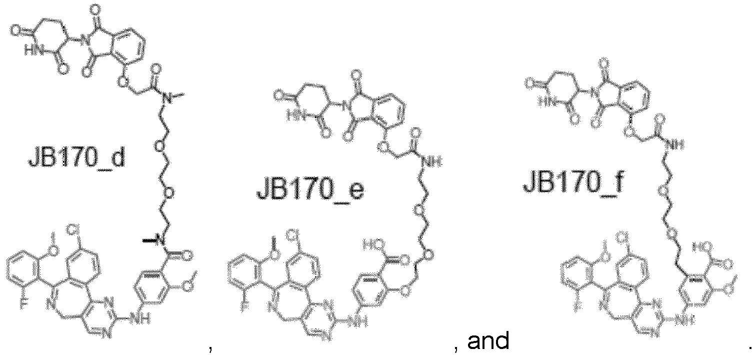

11. Method for synthesizing the PROTAC according to any of the preceding claims, wherein 2-((2-(2,6-dioxopiperidin-3-yl)-1,3-dioxoisoindolin-4-yl)oxy)acetic acid (compound **a**) 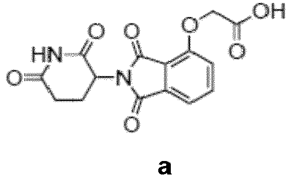 is dissolved in an aprotic solvent followed by addition of (1-[Bis(dimethylamino)methylene]-1H-1,2,3-triazolo[4,5-b]pyridinium 3-oxide hexafluorophosphate (HATU) and N,N-diisopropylethylamine (DIPEA) or trimethylamine as well as a linker precursor having a structure "NH₂-alkylether residue-NH-amine protecting group", "NH₂-polyalkylether residue-NH-amine protecting group", "NH₂-alkyl thioether residue-NH-amine protecting group" or "NH₂-polyalkyl thioether residue-NH-amine protecting group" and further followed by incubation resulting in an intermediate product which is then deprotected to result in compound **b** 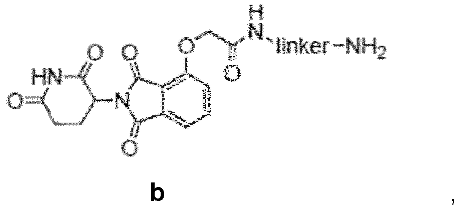 wherein the polyalkyl ether residue has at least two ether groups or at least two ether groups in which at least one O-atom is replaced by an S-atom and the polyalkyl thioether residue has at least two S-atoms, wherein the alkyl ether residue, the polyalkyl ether residue, alkyl thioether residue, or the polyalkyl thioether residue has a chain of atoms having 5 to 17 subsequently arranged atoms, wherein no linear sequence of atoms of the alkyl ether residue, the polyalkyl ether residue, alkyl thioether residue, or the polyalkyl thioether residue exceeds the number of 17 atoms, wherein compound **b** is dissolved in an aprotic solvent followed by addition of (1-[Bis(dimethylamino)methylene]-1H-1,2,3-triazolo[4,5-*b*]pyridinium 3-oxide hexafluorophosphate (HATU), alisertib and N,N-diisopropylethylamine (DIPEA) or trimethylamine and further followed by incubation to result in compound **c**

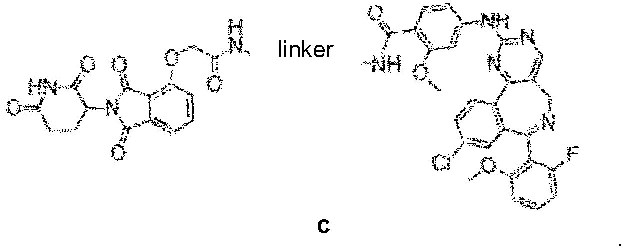

12. Method according to claim 11, wherein the solvent is a polar solvent, in particular Dimethylformamide (DMF), and/or the amine protecting group is tert-butyloxycarbonyl (Boc).

13. Method according to claim 11 or 12, wherein the linker precursor is any of following compounds **1** to **5**:
1. NH₂-CH₂-CH₂-O-CH₂-CH₂-NH-Boc
2. NH₂-CH₂-CH₂-(CH₂-O-CH₂)₃-CH₂-CH₂-NH-Boc
3. NH₂-CH₂-(CH₂-O-CH₂)₂-CH₂-NH-Boc
4. NH₂-(CH₂)₃-NH-Boc
5. NH₂-(CH₂)₅-NH-Boc.

14. PROTAC according to any of claims 1 to 10 for use in the treatment of cancer of a human being or another mammal.

15. PROTAC according to claim 14 for the use according to claim 14, wherein the cancer is leukemia, neuroblastoma, hepatocellular carcinoma or osteosarcoma.
